# FASCICULE DE BREVET EUROPEEN

(11) **EP 1 983 844 B1**
(45) Date de publication et mention de la délivrance du brevet: **15.12.2010**
(21) Numéro de dépôt: 07704603.5
(22) Date de dépôt: 15.02.2007
(51) Int. Cl.: A23L 1/20, A23C 11/10, A61K 36/48

(54) **PROCEDE D'OBTENTION D'EXTRAITS ACTIFS A PARTIR DE GRAINES DE SOJA ET UTILISATIONS DES EXTRAITS OBTENUS CORRESPONDANTES**
VERFAHREN ZUR HERSTELLUNG VON WIRKSTOFFEXTRAKTEN AUS SOJABOHNEN UND ENTSPRECHENDE ANWENDUNGEN DER HERGESTELLTEN EXTRAKTE
METHOD FOR PRODUCTION OF ACTIVE EXTRACTS FROM SOYA BEANS AND CORRESPONDING USES OF THE PRODUCED EXTRACTS

(30) Priorité: 15.02.2006 FR 0601332
(43) Date de publication de la demande: 29.10.2008
(73) Titulaire: SOJASUN TECHNOLOGIES, 35530 Noyal-sur-Vilaine (FR)
(72) Inventeur: EFSTATHIOU, Théo, F-35740 Pace (FR); DRISS, Fathi, F-92160 Antony (FR)
(74) Mandataire: Larcher, Dominique
(86) Numéro de dépôt international: PCT/EP2007/051479
(87) Numéro de publication internationale: WO 2007/093628

(56) Documents cités:
- FR-A- 2 334 308
- JP-A- 11 018 759
- US-A- 2 795 502
- US-A- 2 930 700
- US-A- 3 873 730
- US-A- 3 878 302
- US-A- 4 216 235
- "Food for cancer prevention - prepd. by reacting at least one microbe with an iso-flavone glucoside in soybean milk" DERWENT, 16 septembre 1997 (1997-09-16), XP002228891 & DATABASE WPI Week 199747 Derwent Publications Ltd., London, GB; AN 1997-506520 & JP 09 238647 A (YAKULT HONSHA KK) 16 septembre 1997 (1997-09-16)

## Description

Le domaine de l'invention est celui de la nutraceutique. Plus précisément, l'invention concerne un procédé d'obtention d'un extrait actif de soja destiné à être associé à un produit alimentaire en vue de lui conférer des propriétés anti-oxydantes et/ou anti-inflammatoires et/ou anti-élastase.

La « nutraceutique » se rapporte à tout aliment ou ingrédient alimentaire ayant un bénéfice pour la santé, tel que la prévention ou le traitement d'une maladie.

Plus précisément, la nutraceutique vise les préparations alimentaires qui procurent des bénéfices supérieurs à la nutrition classique, les aliments pour nutrition spécialisée adaptés aux régimes de patients sous conditions médicales particulières comme les patients atteints de diabète, les régimes pour jeunes enfants et nouveau-nés, et maintenant les régimes spécialisés pour personnes âgées, et enfin les compléments alimentaires et plus précisément les compléments nutritionnels médicaux.

Parmi ces compléments nutritionnels médicaux, on peut citer :
- les spécialités permettant de réduire la fréquence des troubles légers en renforçant les défenses naturelles de l'organisme ou en limitant de façon précoce les dérives métaboliques de l'organisme ;
- les spécialités contenant des actifs naturels à efficacité prouvée scientifiquement, permettant de prévenir ou retarder les pathologies reconnues et à forte prévalence, et/ou de réduire les traitements (fréquence et dose) médicamenteux lourds, et/ou de limiter les effets secondaires des traitements et d'améliorer ainsi le bien être des patients.

Le soja, depuis longtemps utilisé en Asie dans l'alimentation traditionnelle, est devenue en Occident une matière première industrielle dans la composition de produits alimentaires.

Dans son exploitation industrielle, c'est principalement sous forme d'ingrédients issus de la graine que le soja est utilisé dans la fabrication d'aliments. Ces ingrédients sont utilisés pour leurs propriétés fonctionnelles (fixation d'eau, liaison, émulsion, texturation, etc) ou leurs propriétés nutritionnelles.

Sur le plan nutritionnel, le soja constitue un apport de nutriments dans l'alimentation, mais peut également être mis à profit dans le cadre d'une nutrition dite « préventive ».

Au niveau industriel, des recherches ont été conduites en vue de démontrer des propriétés de certaines fractions de la graine de soja.

Les composés étudiés ont été extraits des fractions protéique et lipidique, de fibres et en grande partie de molécules spécifiques.

Toutefois, en dépit des nombreuses publications, de nombreux aspects sont encore à explorer concernant le soja.

On sait néanmoins que le soja contient des isoflavones (génistéine, daidzéine, glycétéine) qui font depuis des années l'objet de recherches ciblées. Ces isoflavones ont des propriétés antioxydantes et interviendraient dans diverses actions biologiques associées aux extraits de soja dans le cadre d'une nutrition préventive.

Or, actuellement, les techniques utilisées pour obtenir et étudier des extraits de graines de soja restent à un niveau exploratoire.

Il apparaît donc souhaitable, dans la perspective d'une utilisation croissante du soja alimentaire, de proposer une technique d'obtention d'extraits actifs à partir du soja qui puisse être exploitée industriellement.

C'est l'objectif de l'invention.

Un autre objectif de l'invention est de proposer des utilisations des extraits obtenus à partir du soja.

L'invention a aussi pour objectif de fournir une technique d'obtention d'extraits actifs à partir du soja qui soit simple de conception et facile à mettre en oeuvre.

Ces objectifs, ainsi que d'autres qui apparaîtront par la suite, sont atteints grâce à l'invention qui a pour objet un procédé d'obtention d'au moins un extrait actif riche en manninotriose et en mélibiose à partir de graines de soja, comprenant au moins une étape d'extraction de jus de soja, une étape d'obtention d'un perméat par ultrafiltration dudit jus de soja, préférentiellement une étape de concentration dudit perméat, avantageusement par osmose inverse et/ou évaporation sous vide, le perméat ainsi obtenu étant essentiellement exempt de lipides et de protéines, et au moins une étape d'hydrolyse dudit perméat par voie ° fermentaire réalisée à l'aide d'au moins une levure boulangère en l'absence de tout ferment lactique, étape au cours de laquelle une hydrolyse enzymatique des sucres résultant de l'activité invertase de ladite levure boulangère est effectuée et à l'issue de laquelle ledit extrait actif est obtenu.

Ainsi, on réalise une fermentation particulièrement efficace par la mise en oeuvre d'au moins une levure boulangère.

En, effet, ces levures possèdent une forte activité invertase (β-fructofuranosidase) et sont dépourvues d'activité mélibiase (α-galactosidase). Or, l'invertase, majoritairement exocellulaire, coupe la liaison glucose-fructose β1-2 pour libérer le fructose terminal. Aussi, l'alpha-galactosidase spécifique des liaisons α faisant intervenir un résidu galactose, son absence permet de récupérer en fin de fermentation un extrait riche en mélibiose et manninotriose, issus respectivement de la transformation du raffinose et du stacchyose.

Selon une solution préférée, ladite levure boulangère est *Saccharomyces* cerevisiae par exemple celle commercialisée par la société Lesaffre sous la dénomination *« SAFINSTANT* ».

Ces levures présentent une souche à fermentation haute qui s'avère particulièrement performante dans le contexte de l'invention, son activité invertase (dont l'intérêt a été mentionné précédemment) étant particulièrement forte.

Préférentiellement, ladite étape d'ultrafiltration est mise en oeuvre grâce à au moins une membrane d'ultrafiltration présentant un seuil de coupure compris entre 5000 et 50000 daltons, préférentiellement entre 5000 et 10000 daltons.

Selon une solution avantageuse, ladite étape d'hydrolyse par voie fermentaire est réalisée pendant une durée d'au moins 2 heures, cette durée étant préférentiellement d'environ 2,5 heures.

Avantageusement, le procédé comprend une étape de déshydratation (par exemple par atomisation ou lyophilisation) dudit ou des extraits actifs.

Ainsi, ledit extrait déshydraté présente une teneur en oligosaccharides à galactose terminal avantageusement d'au moins 40% en poids.

Préférentiellement, le procédé comprend au moins une étape de dessalage dudit ou desdits extraits actifs, réalisée préférentiellement à l'aide d'une résine échangeuse d'ions.

Les extraits obtenus peuvent présenter un taux élevé de cendres. L'étape de dessalage permet alors de retenir les anions et les cations et a pour effet de diminuer fortement le taux de minéraux et partiellement celui de MAT et d'isoflavones. Elle permet aussi d'augmenter celui d'oligosaccharides à galactose terminal, qui peut se situer alors jusqu'à environ 85 % de l'extrait sec, en majorité représenté par du manninotriose (environ 90 % de ces 85%) et du mélibiose (environ 10 % de ces 85 %).

Avantageusement, le procédé comprend une étape d'ajout de silice alimentaire audit ou auxdits extraits actifs.

Cet ajout s'avère avantageux dans le contexte de l'invention, le produit dessalé et lyophilisé étant très hygroscopique.

L'invention concerne aussi l'utilisation d'un extrait actif obtenu par un procédé tel que décrit précédemment pour la fabrication d'une composition nutraceutique à propriétés anti-oxydantes.

L'invention concerne aussi l'utilisation d'un extrait actif obtenu par un procédé tel que décrit précédemment pour la fabrication d'une composition nutraceutique à propriétés anti-inflammatoires.

L'invention concerne aussi l'utilisation d'un extrait actif obtenu par un procédé tel que décrit précédemment pour la fabrication d'une composition nutraceutique à propriété anti-élastase.

D'autres caractéristiques et avantages de l'invention apparaîtront plus clairement à la lecture de la description suivante d'un mode de réalisation préférentiel de l'invention, donné à titre d'exemple illustratif et non limitatif, ainsi que de tests réalisés, en référence aux dessins annexés parmi lesquels :
- la figure 1 est une représentation sous forme synoptique d'un procédé selon l'invention ;
- la figure 2 est un diagramme illustrant l'effet dose d'un produit fermenté de soja (PFS) sur des formes réactives de l'oxygène (FRO) par des polynucléaires neutrophiles (PN) au repos ;
- la figure 3 est un diagramme illustrant l'effet dose d'un produit fermenté de soja (PFS) sur des formes réactives de l'oxygène (FRO) par des polynucléaires neutrophiles (PN) stimulées par PMA (phorbol myristate acétate) ;
- la figure 4 est un diagramme illustrant l'effet du PFS sur la production de l'anion superoxyde ;
- la figure 5 est un diagramme illustrant l'effet du PFS sur la dégranulation des PN ;
- la figure 6 est un diagramme illustrant l'effet du PFS sur la production des 02-basal ;
- la figure 7 est un diagramme illustrant l'effet du PFS sur la production des 02 sous PMA ;
- la figure 8 est un diagramme illustrant l'effet du PFS sur la myéloperoxydase des PN stimulés par PMA ;
- la figure 9 est un diagramme illustrant l'effet du PFS sur l'Elastase de porc ;
- la figure 10 est un diagramme illustrant l'effet du PFS sur l'Elastase des PN stimulés par PMA.

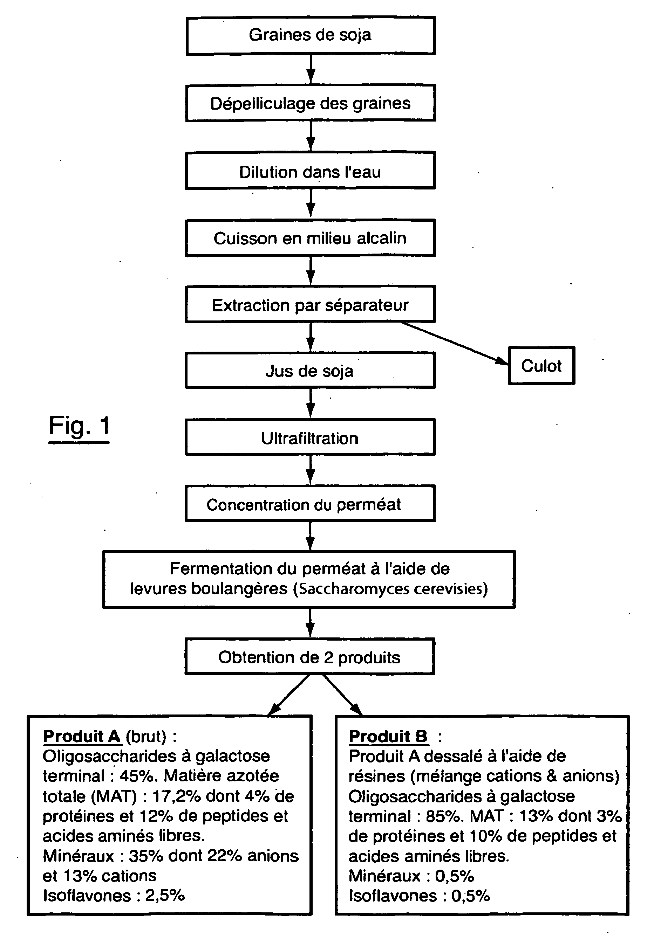

En référence à la figure 1, on fait subir à des graines de soja une succession d'étapes (dépelliculage, dilution, cuisson, extraction, ultrafiltration du jus extrait) en vue d'obtenir un perméat. Ce perméat est préférentiellement concentré par osmose inverse et/ou évaporation sous vide. Ce perméat de soja forme un substrat de départ.

Ce perméat de soja est composé en grande partie de sucres :
- saccharose [α-D-glucose-(1→2)-β-D-fructose],
- raffinose [α-D-galactose-(1→6)-α-D-glucose-(1→2)-β-D-fructose],
- stacchyose [α-D-galactose-(1→6)-α-D-galactose-(1→6)-α-D-glucose-(1→2)-β-D- fructose].

Ces trois sucres possèdent un galactose en position terminale non réductrice, et une extrémité fructose liée à glucose en β1-2.

Afin d'éliminer le résidu fructose sans toucher au résidu galactose pour obtenir le mélibiose et le manninotriose, une hydrolyse par voie fermentaire est réalisée grâce à au moins une levure boulangère, en l'absence de tout ferment lactique.

Les enzymes libérées dans le milieu par les micro-organismes vont permettre de couper la liaison β1-2 entre le glucose et le fructose (1).

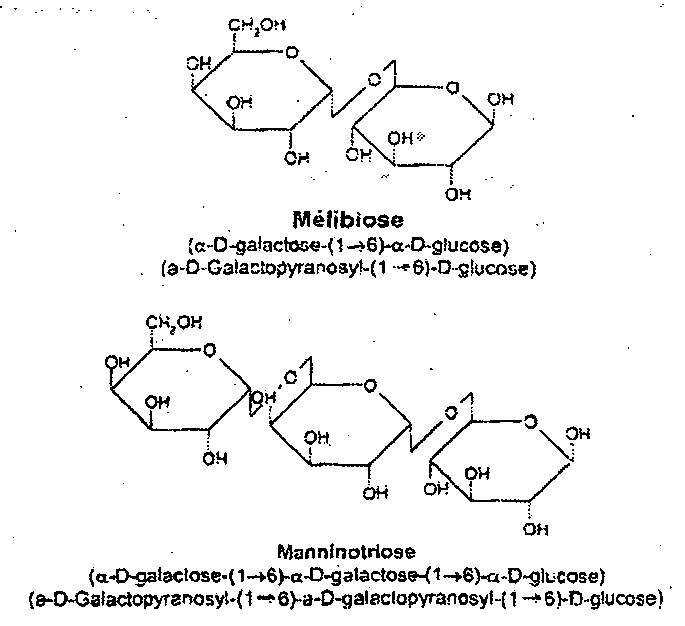

Préférentiellement, la levure boulangère utilisée est *Saccharomyces cerevisiae.*

Dans les conditions mises au point en laboratoire, la concentration maximale en mélibiose et manninotriose est atteinte en 2 heures de fermentation. La durée optimale de fermentation a été fixée à 2,5 heures. La concentration en α-galactocides d'intérêt est alors de 45 g pour 100 g d'extrait sec, en majorité représentés par du manninotriose (90 %) et du mélibiose (10%).

Le produit obtenu est lyophilisé. Il contient un taux élevé de cendres, un dessalage est donc réalisé grâce à une résine mixte échangeuse d'ions AX-X8 (Biorad, marque déposée).

De la silice alimentaire est ensuite ajoutée, à hauteur de 0,2 à 2 % en poids : préférentiellement à hauteur de 1 % en poids, au produit dessalé et atomisé.

Le produit fermenté de soja (PFS) a été testé en vue de mettre en évidence ses propriétés anti-oxydantes, anti-inflammatoires, anti-élastase.

Le produit testé se présente sous la forme de poudre hydrosoluble. Il possède au moins quatre molécules actives : génistéine, daidzéine, manninotriose et mélibiose.

Une concentration efficace a été recherchée en prenant comme référence la molécule de génisteine de PM=270.

Le produit a été dissous dans un tampon PBS pour obtenir une solution de travail correspondant à 1,05 millimolaire (en équivalent génisteine), à partir de laquelle des dilutions permettent de tester des concentrations en milieu final allant de 1 µM à 10 µM.

### 1- Effet du PFS sur la production de formes réactives de l'oxygène (FRO) par les polynucléaires neutrophiles (PN) humains :

Les PN (Polynucléaires neutrophiles) sont isolés du sang total par la technique d'isolement classique basée sur la sédimentation du Dextran/Ficoll.

La mesure de la production des FRO est réalisée par la technique de chimioluminescence amplifliée au luminol en utilisant 500 000 PN par test. Les PN sont préincubés en présence ou absence du produit à 37°C pendant 10 minutes, et la production de FRO est quantifiée grâce à un luminomètre (Berthold 953). La production basale et celle après stimulation par un agoniste (le PMA) ont été mesurées.

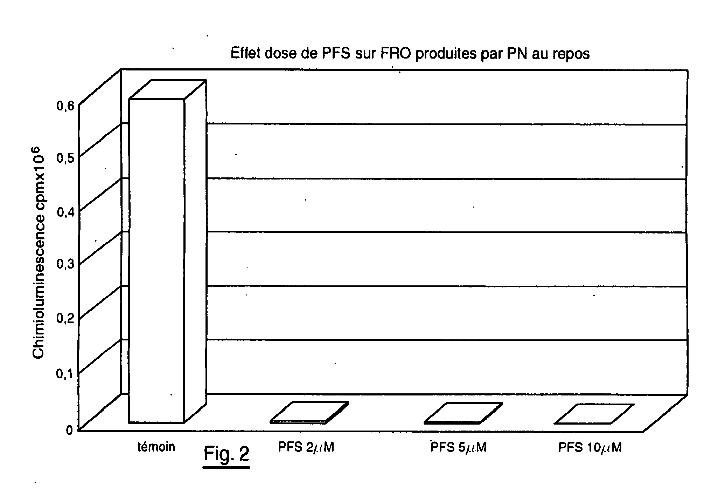

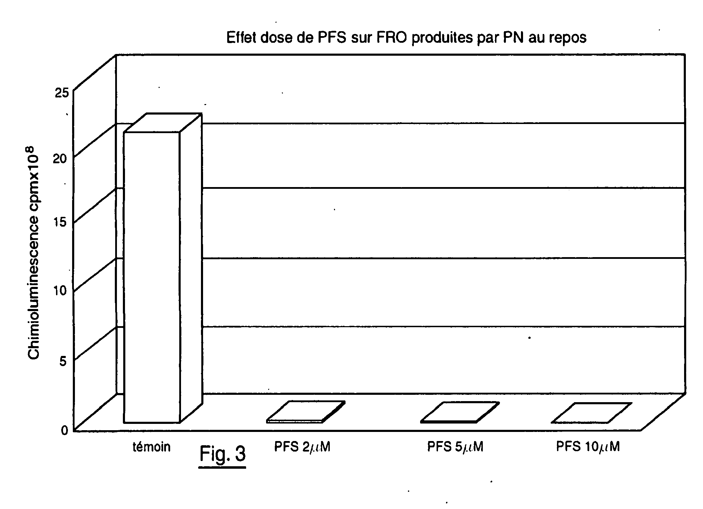

Les résultats sont présentés dans les figures 2 et 3 : le PFS diminue significativement la chimioluminescence induite par PMA de 8,9 x10⁷ cpm (PN contrôle) vs 2.2x10⁷, 1.8x10⁶ et 1.6x10⁶ (pour les PN traités par 0,1 µM, 2 µM et 5 µM PFS respectivement).

### 2- Effet du PFS sur la production de l'anion superoxyde par les polynucléaires neutrophiles (PN) humains :

La stimulation des PN par le PMA active la NADPH oxydase, l'enzyme responsable de la production d'anion superoxyde à l'origine des autres FRO.

La cinétique de cette activation est mesurée par la technique de réduction du Cytochrome C par l'anion 02-, enregistrée grâce à la variation d'absorption à 550 nm. Une unité de DO correspond à 1 nanomole d'O2-produit par minute.

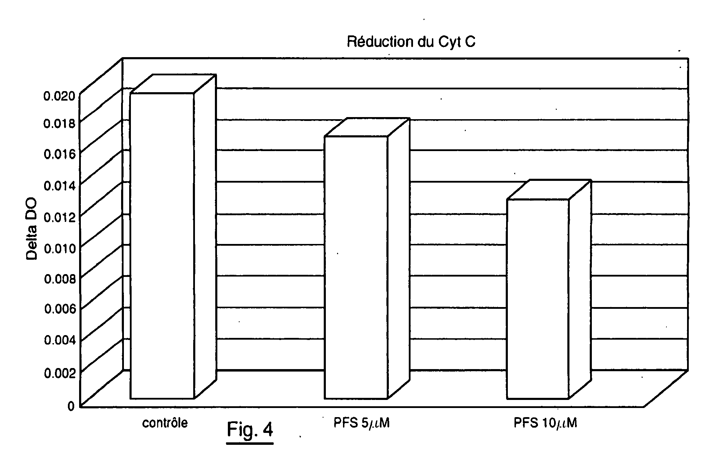

Les résultats de l'effet du PFS sur la production de l'anion superoxyde sont représentés dans la figure 4.

ΔDO=0,02 pour les PN non traités, vs ΔDO=0,017, et ΔDO=0,013 pour les PN traités par le PFS 5 µM et 10 µM respectivement.

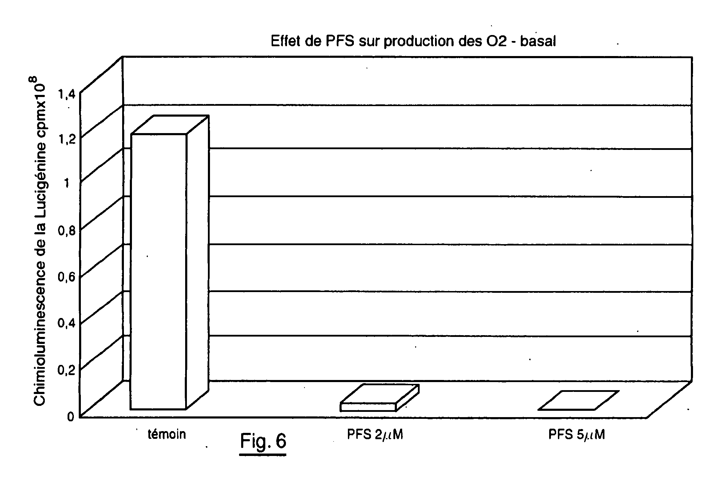

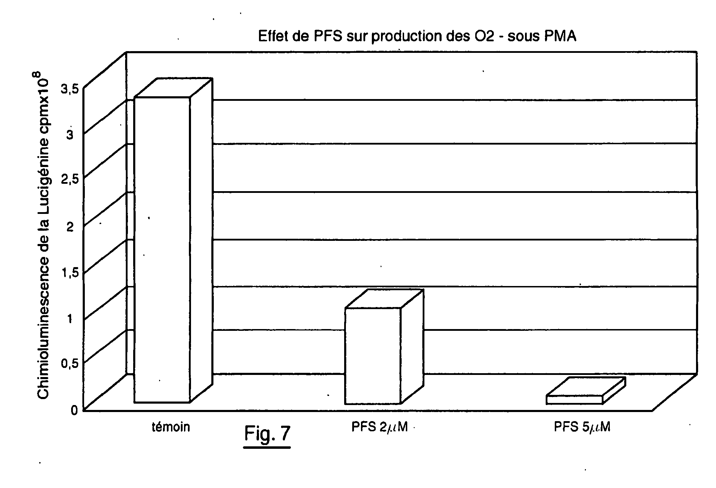

La production de O²⁻ a été mesurée par la chimioluminescence de la Lucigénine sur des PN au repos et après stimulation par la PMA. Le PFS diminue cette production aussi bien à l'état basal (figure 6) qu'après stimulation par le PMA (figure 7) aux concentrations de 2 et 5 µM.

### 3- Effet du PFS sur la dégranulation des PN :

Les PN isolés ont été incubés en présence de PFS à 30°C pendant 10 mn puis stimulés par du PMA pour induire une dégranulation et une libération du contenu des granules dans le surnageant.

L'activité du lysozyme (présent dans les différents types de granules) a été mesurée en utilisant *micrococcus lysodecticus* comme substrat et en enregistrant sa dégradation à 450 nm.

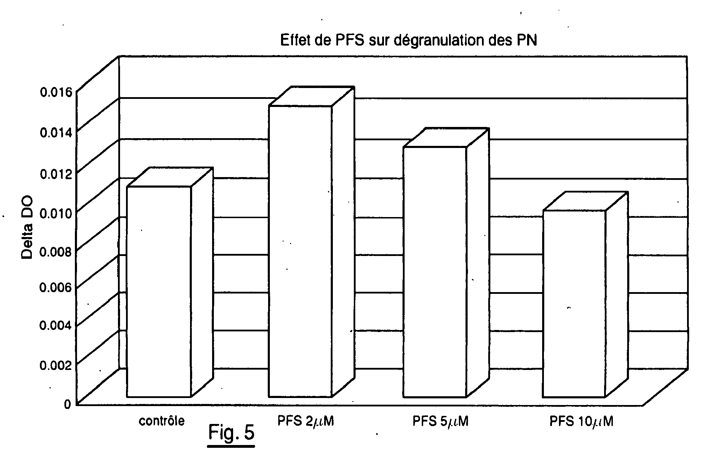

Les résultats montrent que le PFS n'a pas d'effet significatif sur la dégranulation des PN stimulés par le PMA (figure 5).

### 4- Effet du PFS sur l'activité myélopéroxydase des PN :

La myélopéroxydase, située dans les granules azurophiles des PN, est une enzyme clé du métabolisme oxydatif et de la réaction inflammatoire. Elle utilise le péroxyde d'hydrogène (H₂O₂) comme substrat pour produire l'acide hypochloreux (HOCl) hautement toxique.

L'activité myélopéroxydase des PN a été déterminée dans le surnageant des PN stimulés par le PMA utilisant la technique d'oxydation de l'orthodianizidine.

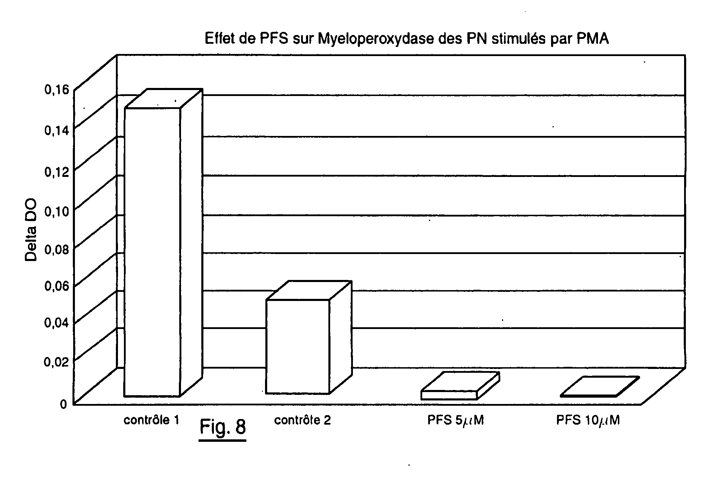

Les résultats montrent que le PFS inhibe cette activité à la concentration de 5 µM et l'abolie complètement à la concentration de 10 µM (figure 8).

### 5- L'effet du PFS sur l'activité Elastase des PN :

L'élastase est une protéase située dans les granules azurophiles des PN ; elle joue un rôle clé dans la réaction inflammatoire et la lutte anti-infectieuse. Son activité est déterminée dans le surnageant des PN stimulés par le PMA en utilisant un substrat spécifique et en suivant sa dégradation à 340 nm.

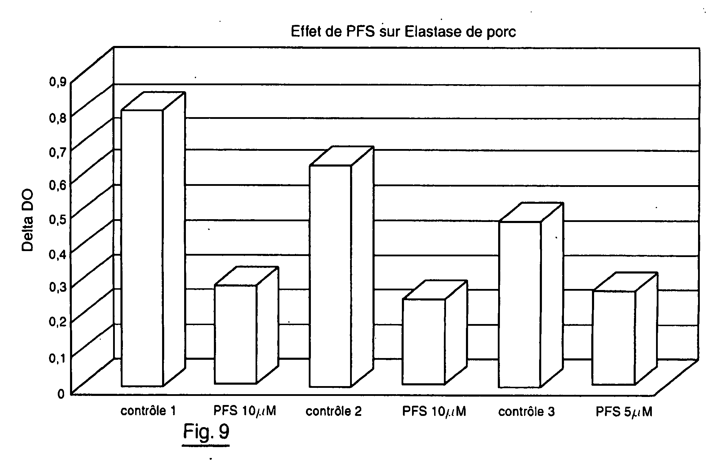

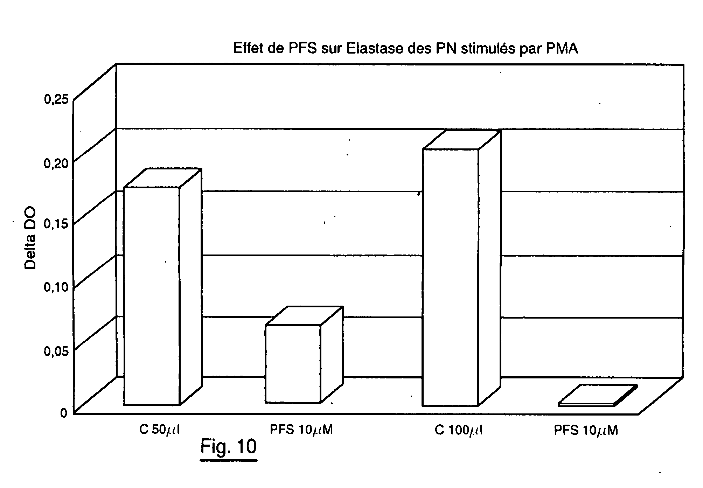

Les résultats montrent que le PFS inhibe fortement et de façon dose dépendante l'activité élastase des neutrophiles (figures 9 et 10).

### 6. Confirmation des propriétés inflammatoires

L'effet du PFS sur la sécrétion de molécules plus spécifiques de l'inflammation, à savoir les cytokines (TNFα) a également été étudié. Pour ce faire, des dosages de cytokine ont été réalisés sur des surnageant de cellules (monocytes, macrophages et polynucléraires). Les résultats de ces dosages ont montré une inhibition de TNFα par le PFS.

### Conclusions

L'ensemble de ces résultats montrent que le PFS inhibe l'activité de la NADPH oxydase et par conséquent la production des FRO et de l'anion superoxyde.

De plus, le PFS diminue l'activité de la myélopéroxydase et de l'élastase des neutrophiles.

Toutes ces enzymes sont impliquées, à la fois dans la défense de l'organisme contre les agents pathogènes, mais aussi dans la réaction inflammatoire induite par de multiples facteurs.

Ces résultats montrent que le PFS est un puissant antioxydant et susceptible de présenter un pouvoir anti-inflammatoire et qu'il présente une activité anti-élastase.

## Revendications

1. Procédé d'obtention d'au moins un extrait actif riche en manninotriose et en mélibiose à partir de graines de soja, comprenant au moins une étape d'extraction de jus de soja, une étape d'obtention d'un perméat par ultrafiltration dudit jus de soja, le perméat ainsi obtenu étant essentiellement exempt de lipides et de protéines, et au moins une étape d'hydrolyse dudit perméat par voie fermentaire réalisée à l'aide d'au moins une levure boulangère en l'absence de tout ferment lactique, étape au cours de laquelle une hydrolyse enzymatique des sucres résultant de l'activité invertase de ladite levure boulangère est effectuée et à l'issue de laquelle ledit extrait actif est obtenu.

2. Procédé selon la revendication 1 **caractérisé en ce qu'**il comprend une étape de concentration dudit perméat par osmose inverse et/ou évaporation sous vide.

3. Procédé selon la revendication 1 ou 2, **caractérisé en ce que** ladite levure boulangère est *Saccharomyces cerevisiae.*

4. Procédé selon l'une quelconque des revendications 1 à 3, **caractérisé en ce que** ladite étape d'hydrolyse par voie fermentaire est réalisée pendant une durée d'au moins 2 heures.

5. Procédé selon la revendication 4, **caractérisé en ce que** ladite étape d'hydrolyse par voie fermentaire est réalisée pendant une durée d'environ 2,5 heures.

6. Procédé selon l'une quelconque des revendications 1 à 5 **caractérisé en ce que** ladite étape d'ultrafiltration est mise en oeuvre grâce à au moins une membrane d'ultrafiltration présentant un seuil de coupure compris entre 5000 et 50000 daltons, préférentiellement entre 5000 et 10000 daltons.

7. Procédé selon l'une quelconque des revendications 1 à 6, **caractérisé en ce qu'**il comprend une étape de déshydratation dudit extrait actif

8. Procédé selon la revendication 7 **caractérisé en ce que** ledit extrait déshydraté présente une teneur en oligosaccharide à galactose terminal d'au moins 40% en poids.

9. Procédé selon l'une quelconque des revendications 1 à 8, **caractérisé en ce qu'**il comprend au moins une étape de dessalage dudit extrait actif

10. Procédé selon la revendication 9, **caractérisé en ce que** ladite étape de dessalage est réalisée à l'aide d'une résine échangeuse d'ions.

11. Procédé selon l'une quelconque des revendications 1 à 10, **caractérisé en ce qu'**il comprend une étape d'ajout de silice alimentaire audit extrait actif.

12. Utilisation d'un extrait actif obtenu par un procédé selon l'une quelconque des revendications 1 à 11, pour la fabrication d'une composition nutraceutique à propriétés anti-oxydantes.

13. Utilisation d'un extrait actif obtenu par un procédé selon l'une quelconque des revendications 1 à 11 pour la fabrication d'une composition nutraceutique à propriétés anti-inflammatoires.

14. Utilisation d'un extrait actif obtenu par un procédé selon l'une quelconque des revendications 1 à 11, pour la fabrication d'une composition nutraceutique à propriété anti-élastase.

## Claims

1. Method for obtaining at least one active extract which is rich in manninotriose and melibiose from soya beans, comprising at least one step for extracting soya juice, a step for obtaining a permeate by ultrafiltration of the said soya juice, the permeate obtained in this manner being substantially free from lipids and proteins, and at least one step for hydrolysis of the said permeate via fermentation carried out using at least one baker's yeast without any lactic ferment, a step during which an enzymatic hydrolysis of the sugars resulting from the invertase activity of the said baker's yeast is carried out and following which the said active extract is obtained.

2. Method according to claim 1, **characterised by** a step for concentrating the said permeate by reverse osmosis and/or vacuum evaporation.

3. Method according to claim 1 or claim 2, **characterised in that** the said baker's yeast is *Saccharomyces cerevisiae.*

4. Method according to any one of claims 1 to 3, **characterised in that** the said step for hydrolysis via fermentation is carried out for a duration of at least 2 hours.

5. Method according to claim 4, **characterised in that** the said step for hydrolysis via fermentation is carried out for a duration of approximately 2.5 hours.

6. Method according to any one of claims 1 to 5, **characterised in that** the said ultrafiltration step is carried out using at least one ultrafiltration membrane which has a molecular weight cutoff of between 5000 and 50000 daltons, preferably between 5000 and 10000 daltons.

7. Method according to any one of claims 1 to 6, **characterised in that** it comprises a step for dehydration of the said active extract.

8. Method according to claim 7, **characterised in that** the said dehydrated extract has a content of oligosaccharides with terminal galactose of at least 40% by weight.

9. Method according to any one of claims 1 to 8, **characterised in that** it comprises at least one step for desalination of the said active extract.

10. Method according to claim 9, **characterised in that** the said desalination step is carried out using an ion-exchange resin.

11. Method according to any one of claims 1 to 10, **characterised in that** it comprises a step for adding food silica to the said active extract.

12. Use of an active extract obtained using a method according to any one of claims 1 to 11, for producing a nutraceutical composition having anti-oxidant properties.

13. Use of an active extract obtained using a method according to any one of claims 1 to 11, for producing a nutraceutical composition having anti-inflammatory properties.

14. Use of an active extract obtained using a method according to any one of claims 1 to 11, for producing a nutraceutical composition with anti-elastase property.

## Patentansprüche

1. Verfahren zur Herstellung von zumindest einem aktiven Extrakt, reich an Manninotriose und an Melibiose, aus Sojabohnen, umfassend zumindest einen Schritt der Extraktion von Sojasaft, einen Schritt zum Erhalten eines Permeats durch Ultrafiltration des Sojasaftes, wobei das so erhaltene Permeat im Wesentlichen frei von Lipiden und Proteinen ist, und zumindest einen Schritt der Hydrolyse des Permeats durch Fermentation mit Hilfe von wenigstens einer Bäckerhefe in Abwesenheit von jeglichem Milchsäureferment, ein Schritt, während dessen enzymatische Hydrolyse von Zuckern, resultierend aus der Invertaseaktivität der Bäckerhefe, durchgeführt wird und nach dem der besagte aktive Extrakt erhalten wird.

2. Verfahren nach Anspruch 1, **dadurch gekennzeichnet, dass** es einen Schritt zur Konzentration des besagten Permeats durch inverse Osmose und/oder Vakuumverdampfung umfasst.

3. Verfahren nach Anspruch 1 oder 2, **dadurch gekennzeichnet, dass** die Bäckerhefe *Saccharomyces cerevisiae* ist.

4. Verfahren nach einem der Ansprüche 1 bis 3, **dadurch gekennzeichnet, dass** der besagte Schritt der Hydrolyse durch Fermentation über einen Zeitraum von mindestens 2 Stunden durchgeführt wird.

5. Verfahren nach Anspruch 4, **dadurch gekennzeichnet, dass** der besagte Schritt der Hydrolyse durch Fermentation über einen Zeitraum von ca. 2,5 Stunden durchgeführt wird.

6. Verfahren nach einem der Ansprüche 1 bis 5, **dadurch gekennzeichnet, dass** der besagte Schritt der Ultrafiltration mit zumindest einer Ultrafiltrationsmembran, die einen Cut off aufweist zwischen 5000 und 50000 Dalton, vorzugsweise zwischen 5000 und 10000 Dalton, durchgeführt wird.

7. Verfahren nach einem der Ansprüche 1 bis 6, **dadurch gekennzeichnet, dass** es einen Schritt der Dehydratation des besagten aktiven Extraktes umfasst.

8. Verfahren nach Anspruch 7, **dadurch gekennzeichnet, dass** der besagte dehydratisierte Extrakt einen Gehalt an Oligosacchariden mit terminaler Galactose von zumindest 40 Gew.-% aufweist.

9. Verfahren nach einem der Ansprüche 1 bis 8, **dadurch gekennzeichnet, dass** es zumindest einen Schritt zur Entsalzung des besagten aktiven Extraktes umfasst.

10. Verfahren nach Anspruch 9, **dadurch gekennzeichnet, dass** der besagte Schritt zur Entsalzung mit Hilfe eines Ionenaustauscherharzes durchgeführt wird.

11. Verfahren nach einem der Ansprüche 1 bis 10, **dadurch gekennzeichnet, dass** es einen Schritt des Hinzufügens von alimentärem Siliziumdioxid zu dem besagten aktiven Extrakt umfasst.

12. Verwendung eines aktiven Extraktes, erhalten durch ein Verfahren nach einem der Ansprüche 1 bis 11, für die Herstellung einer nutrazeutischen Zusammensetzung mit antioxidativen Eigenschaften.

13. Verwendung eines aktiven Extraktes, erhalten durch ein Verfahren nach einem der Ansprüche 1 bis 11, für die Herstellung einer nutrazeutischen Zusammensetzung mit antiinflammatorischen Eigenschaften.

14. Verwendung eines aktiven Extraktes, erhalten durch ein Verfahren nach einem der Ansprüche 1 bis 11, für die Herstellung einer nutrazeutischen Zusammensetzung mit Antielastase-Eigenschaft.
